# EUROPEAN PATENT APPLICATION

(11) **EP 2 272 471 A1**
(43) Date of publication of application: **12.01.2011**
(21) Application number: 10169064.2
(22) Date of filing: 09.07.2010
(51) Int. Cl.: A61F 5/01, B29C 67/00

(54) **An artificial exoskeleton device or an orthotic device comprising an integrated hinge structure**

(30) Priority: 09.07.2009 GB 0911953
(71) Applicant: Materialise NV, 3001 Leuven (BE)
(72) Inventor: Pallari, Jari Heikki Petteri, B-3000, Leuven (BE); Wirix-Speetjens, Roel, B-3680, Maaseik (BE)
(74) Representative: Bird, William Edward

(57) **Abstract**

A pivot or hinge structure integrated into the main body of an exoskeletal, e.g. orthotic device is described as well as methods for computer aided designing and making of these devices. Computer systems and software for carrying out the methods are also described.

The integrated pivot or hinge structure has a specified axis of rotation, such as one coinciding or approximating with the natural rotation axis of the ankle, knee, elbow or any other relevant joint. Preferably the pivot or hinged structure is provided with a personalised resistance to rotation. The pivot or hinge structure is a pivot or hinge comprising at least two separate cylindrical parts that rotate with respect to each other, the axis of rotation being at the centre of the cylinder. The pivot or hinge can be placed to the optimal location and orientation in regard to the limb or anatomical features observed or measured from the patient thanks to the layered manufacturing technique. The range of motion can be structurally limited if needed. This enables more possibilities for adjustment when the exoskeletal device, e.g. orthotic device is fitted to the patient to ensure a better fit and functionality.

## Description

### FIELD OF THE INVENTION

The present invention relates generally to the field of artificial exoskeletal devices and/or machines, such as orthotic devices and, more specifically, to artificial exoskeletal devices and/or machines, such as orthotic devices having a hinge structure integrated into the main body of the artificial exoskeleton device and/or machines, or orthosis as well as methods for computer aided designing and making of these devices. The present invention also relates to computer systems and software for carrying out the methods

### BACKGROUND TO THE INVENTION

An artificial exoskeleton device is a device that mimics functionally at least part of an external skeleton that supports and/or protects an animal's body, particularly for animals such as human beings who only have an endoskeleton. Exoskeletal machines (also called powered exoskeletons) are used for medical and industrial purposes. Orthoses are a medical form of exoskeleton.

Known exoskeletal devices and machines tend to be made of bulky materials such as frame structures that are only poorly adaptable to the individual human body. Exoskeletal devices or machines hence tend to be bulky and are often worn outside of the clothing.

An orthosis is a device which attaches to a limb, or the torso, to support the function or correct the shape of that limb or the spine. Orthotics is the field dealing with orthoses, their use, and their manufacture. An orthosis is an external device or brace designed to support or assist in the support of a patient, e.g. in carrying the loads applied onto them by walking, running, manipulating objects and/or similar activities and/or by repositioning a limb or forcing them to move a certain way. They can also control joint rotations, such as limiting the rotations to around a certain axis or to a certain range for the ankle joint. The joint can also be immobilized in certain pathologies.

A prosthesis differs from an orthosis. It is a device that, e.g. substitutes for a missing part of a limb. A prosthetic device is an artificial extension of the human body, meant to replace a lost limb or any other body part. Prosthetics is the field that deals with prostheses, their use, and their manufacture. As prosthetics replace a part of the body there is freedom to select designs and materials as appropriate. On the other hand orthotics must complement the existing body parts causing the least discomfort and maximising the aesthetic appeal while still providing functional support and allowing a safe and pain-free range of movements. Hence orthotics pose problems that are not shared with prosthetics.

Currently the design and manufacture of customized orthoses is a multi-stage and labour intensive process with significant elements of clinical judgement, manufacturing craftsmanship and trial-and-error experimentation. Only a few standardized procedures exist in their design and manufacturing. This can lead to variation in the final product. The lengthy manufacturing process can also delay treatment.

Traditionally, manufacturing processes for orthoses include, initially, that a plaster cast of the relevant parts of the limb is taken; this is then worked into a positive of the limb, wherein certain interventions are applied by the craftsman manually. The modified positive is then vacuum formed or laminated using thermo-formable plastic. This device is then further modified, finished and fitted to the patient. Further manual modifications may be necessary, especially when adding hinges to orthoses.

This process is completely manual, requiring considerable experience and skill. Each device is also unique, as the work stages are done slightly differently each time. Also, if several persons are working on the same device, each person has a different idea of what is required.

The need for time-consuming manual tasks makes the overall process slow and specialist equipment and supplies are required. If the design is incorrect, it can occur that the whole process has to be restarted. A computer assisted process may alleviate some of these issues, e.g. those related in creating the positive and making the interventions to it, but adds more process steps in the orthoses creation chain and adds extra investment in training, equipment, milling materials - which also create a lot of waste - without solving the problems with traditional manufacturing completely. The lamination/vacuum forming will still have to be done manually, as will the addition of features such as cushioning, hinges, cutouts, etc. Furthermore, if the orthosis is equipped with a hinge, this will further add to the complexity of the device and to the time it takes to make it. The orientation of the hinge and the location of the rotational axis are up to the individual craftsman resulting in highly variable outcomes.

In Patent WO 95/32691 an adjustable hinge for orthotic applications is described. A hinge with a coil spring is placed between the upper and lower half of the device. It can be adjusted by disconnecting the other end of the spring and connecting it back to another slot in the hinge. The spring can also be replaced. The location of the hinge and thus the center of rotation are up to the craftsman making the orthosis. The structures presented in the above patent cannot be seamlessly adjusted.

It is also known from the prior art, as described in the article in Volume 55(2) (2008) of the IEEE Transactions on Biomedical Engineering to Faustini entitled "Manufacture of passive dynamic ankle-foot orthoses using selective laser sintering", that customized orthotic or prosthetic devices may be manufactured using Selective Laser Sintering (SLS), a Rapid Prototyping and Manufacturing (RP&M) technique, where RP&M can be defined as a group of techniques used to quickly fabricate a scale model of an object typically using 3-D computer aided design (CAD) data of the object.

The devices presented in this paper cannot be adjusted. Also, the type of AFO presented contains no hinges and is used to treat different types of pathologies than hinged ones. Furthermore, the deforming structure here cannot be used to control the rotation in prosthetic joints. To control the ankle rotation precisely, a hinged device is needed. It is not obvious from this presentation how deformable shell structures can be applied to a rotational hinge structure.

Accordingly, those skilled in the art of exoskeletal device or machine, or orthotic device manufacturing and the like recognize the desirability for integrated features and the manufacturing method for orthoses enabling the advantages of the prior art procedures, yet at the same time avoiding at least one of the limitations associated therewith. Also there is a need to provide close fitting devices that are ideally customised to the wearer and yet also optimise the exosketal properties and functionality of the devices to the properties and functionality of the endoskeleton they replace or surround.

### SUMMARY OF THE INVENTION

An object of the present invention is provide exoskeletal, e.g. orthotic devices and, more specifically, exoskeletal, e.g. orthotic devices having a better hinge structure.

The present invention provides a pivot or hinge structure integrated into the main body of the exoskeletal, e.g. orthotic device as well as methods for computer aided designing and making of these devices. The present invention also provides computer systems and software for carrying out the methods.

In one aspect, the present invention provides an exoskeletal, e.g. orthotic device comprising at least one integrated pivot or hinge structure with a specified axis of rotation, such as one coinciding or approximating with the natural rotation axis of the ankle, knee, elbow or any relevant joint. Preferably the pivot or hinged structure is provided with a personalised resistance to rotation. The pivot or hinge structure is a pivot or hinge comprising at least two separate cylindrical parts that rotate with respect to each other, the axis of rotation being at the centre of the cylinder. The pivot or hinge can be placed to the optimal location and orientation in regard to the limb or anatomical features observed or measured from the patient thanks to the layered manufacturing technique. The range of motion can be structurally limited if needed. This enables more possibilities for adjustment when the exoskeletal device, e.g. orthotic device is fitted to the patient to ensure a better fit and functionality.

The above device can be advantageously included in lower extremity orthoses (e.g. ankle and/or knee and/or hip orthoses), upper extremity (e.g. shoulder and/or elbow and/or wrist and/or finger orthoses). Applications of the present invention include braces or splints as well as more complex devices. A primary application is an ankle and/or foot orthotic (AFO application) in which the pivot or hinge structure is integrated in the orthotic shape so that it forms an integrated single piece or assembly, preferably manufactured as a single piece or assembly. Preferably, the manufacture is by layer manufacturing. This provides the advantage that the hinge structure can be scaled up or down in size or built to conform to any 3D shape as necessary and/or blended into or onto the orthotic shape. Ideally this 3D-shape of the hinge structure should include a dished, e.g. a spherical approximation of the ankle. To allow rotation of the hinge the dished shape is preferably a surface of rotation of which the surface of sphere, of a parabola, of an ellipse are only examples.

A resistance to rotation can be achieved by connecting the upper and lower halves with elastically or plastically deformable structures such as spiral and/or coil springs. The main hinge structure carries the majority of the vertical loads together with the patients limb - in an orthotic application - and prevents unnecessary motion and the deforming structures provide the required resistance to motion. Furthermore, in this invention the hinge and the adjustment structures are built as one part, as enabled by layer manufacturing, and as such offer several advantages over traditional manufacturing. Furthermore, the hinge structure in this invention does not have to lay flat on a plane, but can be contoured along a round, flat or elongated sphere or any combination of these shapes. This is beneficial as the centre of rotation of the hinge can be placed very close to the ankle of the patient. The closer the hinge is to the joint, the more natural the motion will be. Also, these possibilities will enable the appearance of the orthoses to be different allowing for more possibilities for product design and personalization.

In embodiments of the present invention an exoskeletal device or an orthotic device is provided comprising a first and a second part, the first part being joined to the second part by a pivot structure which allows pivotal rotational movement of the first part with respect to the second part, wherein the pivot structure is formed integrally with the first and second part by a layered manufacturing technique such as a rapid prototyping technique. Devices according to the present invention can be formed as a shell for encompassing a limb of an animal or human.

The present invention provides the advantage that the hinge structure can be optimally adapted in its shape to various parts of the body such as the ankle or the elbow for which a dished shaped hinge structure is preferred, i.e. fits more closely and can be more comfortable. This allows a close fitting exoskeletal device. It also allows a device that can be adapted to the patient or wearer of the orthosis.

Preferably, means for limiting the angular rotational movement of the first part with respect to the second part can be provided. This allows restraint of the rotation when this is medically advantageous. Preferably, the means for limiting is formed integrally with the pivot structure by the layered manufacturing, e.g. rapid prototyping technique. This provides a convenient way of building in the restraint without requiring extra steps.

For some applications it is advantageous to provide means for providing a resilient force resisting rotation of the first part with respect to the second part. This can allow a more controlled rotation, e.g. when the necessary muscle power is lacking.

Again it is preferably, if the means for providing a resilient force is formed integrally with the pivot structure by the layered manufacturing, e.g. rapid prototyping technique. This provides a convenient way of including this feature without extra steps of adding springs or other resilient devices. For example, the means for providing a resilient force can be a connection between the first and second parts having an elastically or plastically deformable structure. This is a convenient and simple mechanical form that can be provided using a layered manufacturing technique. For example, the elastically or plastically deformable structure can be in the form of a spiral or coil.

To allow for adaptation to patient specific parameters, it is preferred if the means for providing a resilient force is adjustable.

In embodiments of the present invention, the pivot structure is a hinge. Various adjusting devices can be included in the hinge. In preferred embodiments, the pivot structure has a centre of pivoting about which the first and second parts can rotate with respect to each other, a surface of the pivot structure having a shape that deviates from planar, the surface being a surface of rotation, the centre of the surface of rotation coinciding with the centre of pivoting.

The present invention also includes a computer based method of forming an orthotic device for a patient, the computer for example having a processor and memory which are used to carry out the method, the method comprising the steps of:
receiving scan data describing at least part of the patient's body;
converting said scan data into a 3D virtual model on the basis of which a 3D design model of the orthotic device is constructed;
adding automatically or interactively at least one pivot structure from a library of pivot structures, the pivot structure having a centre of pivoting about which the first and second parts can rotate with respect to each other, a surface of the pivot structure having a shape that deviates from planar, the surface being a surface of rotation, the centre of the surface of rotation coinciding with the centre of pivoting;
and
fabricating the orthotic device.

The adding step can include adding an adjustment mechanism for adjusting at least one meaningful property of said pivot structure from a library of adjustment structures.

The method may further comprise:
manufacturing a first and a second part of the orthotic device,
manufacturing the pivot structure so that the first part being is joined to the second part by the pivot structure which allows pivotal rotational movement of the first part with respect to the second part, the pivot structure being formed integrally with the first and second part by a rapid prototyping technique.

The present invention also includes a computer program product comprising code segments, the code segments comprising, when executed on a computing device, e.g. having a processor and memory:
means for receiving scan data describing at least part of the patient body;
means for converting said scan data into a 3D virtual model on the basis of which a 3D design model of the orthotic device may be constructed; and
means for adding automatically or interactively at least one pivot structure and an adjustment mechanism enabling means for adjusting at least one meaningful property of said pivot structure from a library of pivot and adjustment structures.

The present invention also includes a non-transitory signal storage means which stores the computer program product. The means can be an optical disk such as a CD-ROM, a DVD-ROM etc, a tape memory device, a disk memory device such as a hard disc or a diskette, a solid state memory such as a USB stick etc.

### BRIEF DESCRIPTION OF THE DRAWINGS

Embodiments of the invention will be described, by way of example only, with reference to the accompanying drawings in which:
FIG. 1 shows a block diagram for designing and manufacturing an orthotic device comprising at least one hinge structure.
FIG. 2 shows an ankle foot orthoses and the placement of the hinge structure in said orthoses. Also a simplification of the hinge structures according to embodiments of the present invention is shown in figures that follow.
FIG. 3 shows how the axis of rotation for the hinge can be defined.
FIG. 4 shows how a hinge structure to be produced with additive fabrication methods should be designed according to an embodiment of the present invention.
FIG. 5 shows how another kind of hinge structure to be produced with additive fabrication methods should be designed according to an embodiment of the present invention.
FIG. 6 shows yet another type of hinge structure according to an embodiment of the present invention.
FIG. 7 shows how the range of rotation can be structurally limited for the hinge according to an embodiment of the present invention.
FIG. 8 shows one means of adjusting the range of motion on a hinge according to an embodiment of the present invention..
FIG. 9 shows a basic beam hinge structure and defines the cross section of said beam according to an embodiment of the present invention..
FIG. 10 shows different spring structures based on a beam and a spiral spring according to an embodiment of the present invention..
FIG. 11 and12 show further embodiments comprising of deforming structures resisting rotation around the hinge.
FIG. 13 and 14 show further embodiments comprising curved surfaces for the hinge structures.
FIG. 15 illustrates a computer based system for use with embodiments of the present invention.

### DESCRIPTION OF PREFERRED EMBODIMENTS

In the following detailed description of the preferred embodiments, reference is made to the accompanying drawings, which form a part hereof, and within which are shown by way of illustration specific embodiments by which the invention may be practiced. The drawings described are only schematic and are non-limiting. In the drawings, the size of some of the elements may be exaggerated and not drawn on scale for illustrative purposes. Those skilled in the art will recognize that other embodiments may be utilized and structural changes may be made without departing from the scope of the invention.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B.

In the following and in the attached claims reference will be made to a "patient". It should be understood that this term "patient" should be construed broadly to include not only humans but also animals.

The present invention will mainly be described with reference to an ankle foot orthosis (AFO) that is usually a plastic brace for attaching to the calf of the subject with an removable and/or adjustable attachment means such as a strap like a Velcro strap or lacing and with a sole part, fitting under the foot which in some cases can optionally fit inside a shoe. The purpose of an AFO is to control the ankle joint rotations and possibly carry some of the forces applied through the foot and ankle. An AFO can also absorb some of the forces by elastically deforming in response to forces that are placed on it when the user puts his/her weight on it and then releases this energy when the user pushes themselves forward, assisting this movement. The present invention is not limited to AFO but finds advantageous use with a variety of orthotics. It should be understood that the subject matter of this application is also applicable to any other orthotic device, including, but not limited to, knee orthoses, knee-ankle-foot orthoses, hip orthoses, hip-knee-ankle-foot-orthosis, lumbar orthoses, adaptors or joints or cranial helmets. Embodiments of the present invention may be included within artificial exoskeletal devices and/or machines, orthotic devices and, more specifically, in artificial exoskeletal devices and/or machines, orthotic devices having a pivot or hinge structure integrated into the main body of the artificial exoskeleton device and/or machines, or orthosis as well as in methods for computer aided designing and making of these devices.

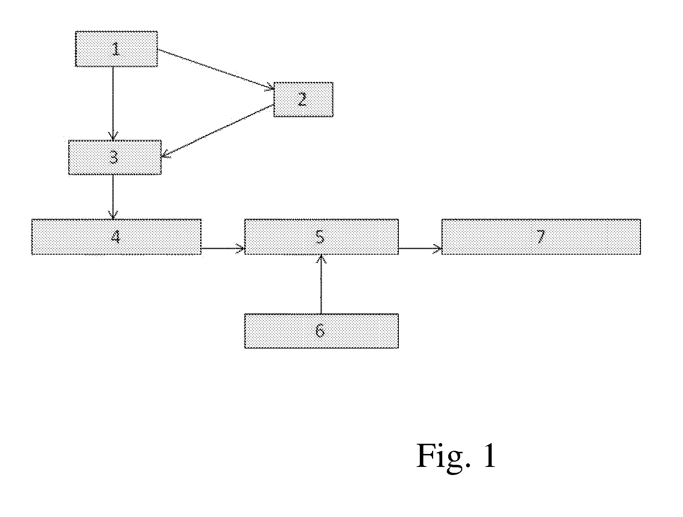

Referring now to the drawings, in particular FIG. 1, there is shown a block diagram that illustrates a preferred embodiment for designing and fabricating an orthotic device according to the present invention. First, the patient, illustrated by numeral 1 in FIG. 1, is sent to a scanning facility with equipment for capturing either directly or indirectly a 3D image of the target surface, e.g. the limb, the ankle and the foot in case of an AFO. The 3D image of the surface may be obtained by any suitable method such as, by a direct non-contact scanning of the object, e.g. by optical scanning, by stereoscopic cameras or by any other suitable optical technique or by contact scanning of the object, e.g. a digitising pen or probe or the 3D surface can be derived from other types of images such as volumetric images generated by techniques such as MRI, CT-Scan, ultrasound, computer tomography, with which the images can be segmented to show surfaces by any suitable technique, e.g. thresholding, "marching cubes", etc. The scanning equipment in module 3 generates a digitized data file that provides data describing the target surface. Next, in module 4 the scanned data may be imported into a computer system running a computer program to show, render or convert the scanned data into a 3D virtual model of the patient's limb. Alternatively, a cast of the limb may be taken, as in module 2, which can then be scanned in module 3 by a scanning equipment. As above the 3D image of the surface may be obtained by any suitable method such as, by a direct non-contact scanning of the cast, e.g. by optical scanning, by stereoscopic cameras or by any other suitable optical technique or by contact scanning of the cast, e.g. using a digitising pen or probe or the 3D surface can be derived from other types of images such as volumetric images generated by techniques such as MRI, CT_Scan, ultrasound, computer tomography, with which the images can be segmented to show surfaces by any suitable technique, e.g. thresholding, "marching cubes", etc.. Once a virtual 3D model of the limb is constructed, a 3D model of the orthoses may be designed based on the 3D model of the limb, as indicated by module 5. This design of the 3D model of the orthoses may be produced interactively or fully computer-controlled. As module 6 indicates, a library system of mechanical structures useful in orthotics such as hinge structures may be used in the design in order to incorporate these structures in the 3D computer model of the orthoses. Once the design is complete, it can be manufactured with a layer fabrication process, as in module 7. For example a descriptor file may be generated and exported to module 7, where the file is received and converted into layer suitable for driving a layer manufacturing equipment.

The limb has always a certain shape, which is preferably to be captured accurately in order to create a well-fitting orthoses. The geometry can be captured non-weight bearing or weight bearing through glass or clear palstic or other such transparent materials, which are pressed against the limb. Alternatively, the patient can stand on a transparent plate and the weight bearing 3D shape captured through the plate. The geometry of the limb can be captured using the following means but not excluding other means of capturing it. In module 1 in FIG 1, Laser scanning technology may be used to generate a digital 3D geometry of the limb shape. This can be in the form of a point cloud, a solid surface consisting of triangles or any other format for recording and storing a 3D geometry. Another way of obtaining the geometry is to manually make a plaster cast of the limb and to use the technique described above to capture the shape of the cast. Alternatively, a positive made from the cast can be scanned. Making casts is how the "traditional" process works and to be able to accommodate this way of working is beneficial and the benefit is obvious to anyone skilled in the art. Apart from laser scanning, capturing the 3D geometry of the limb may be done by means of radiation, e.g. X-rays or ultrasound or through computer tomography (CT) scans or magnetic resonance imaging (MRI) or any other scanning method known to generate medical volumetric data.

The geometry of the limb determined in module 1 can be digitally imported to a computer program and may be converted using algorithms known from the field of CAD/CAM technology to produce a 3D computer model of the limb. A computer program such as 3-matic™ as supplied by Materialise N.V., Leuven, Belgium, may be used for constructing this 3D model. This geometry data can be used immediately in the computer program or stored in a digital file.

Once the 3D model of the limb is constructed, it may be manipulated manually, semiautomatically or automatically to design a 3D model of the orthotic device. These manipulations may include any one or more of the following (but are not limited thereto):
1. Scaling the geometry smaller or larger along certain axis.
2. Defining a contour of the edge of the orthotic as is known by a person skilled in the art.
3. Giving the geometry a thickness that can be varied throughout the part.
4. In creating hollow volumes inside this thickness.
5. Adding new surface shapes in certain parts, such as local elevations.
6. Adding predetermined 3D elements from a database system (E), such as deformable hinge structures.
7. Integrating the interventions made into an optimal orthotic shape.
8. Adding attachment features that enable the attachment of straps or other means to fasten the orthotic device to the person using it and/or other external components, such as reinforcements (carbon fibre, steel, plastic or any other relevant material) and/or electronic devices, such as step counters or any other electronic devices.
9. Adding holes or other features for ventilation purposes.
10. Dividing the orthotic device into several parts if necessary if required by the production technique, size of the machine used for manufacturing or a better utilisation of the build volume of the machine.

A preferred method for performing these actions uses a computer program such a 3-matic as supplied by Materialise N.V., Leuven, Belgium.

A data base library of one or more 3D models of hinge structures or their mathematical representations may then be used to incorporate at least one hinge structure into the 3D model of the orthotic device. The elements in the library may be selected manually or automatically from the database by their pre-determined properties, such as their physical dimensions, their appearance or their mechanical properties, e.g. stiffness, the spring coefficient (for rotational springs), range of motion, crack formation and crack propagation. It is to be understood that the dimensions and values regarding the performance of all hinge structures available in the library may be scaled in any dimension to obtain the preferred or expected mechanical properties and performance. Functions representing them and their performance are stored in this data base so that they can be called when required, automatically or manually by the user, and integrated into the 3D design of the orthoses using the design software. Specific structures may be called from the library or all structures matching certain performance parameters for the user to select for a particular location and purpose.

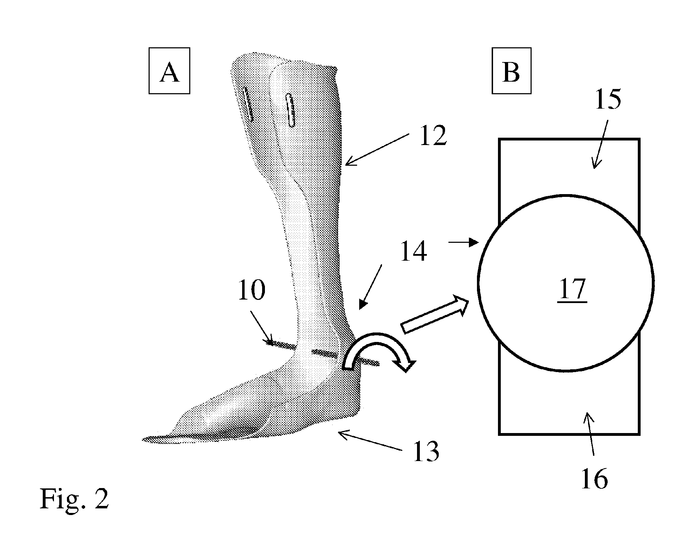

In one embodiment, the pivot or hinge structure is integrated to an Ankle Foot Orthoses (AFO) presented in FIG 2A. The pivot or hinge structure 14 can be placed in (i.e. manufactured into) the orthosis at a position around the ankle such that the hinge axis approximates that of the natural rotation axis of the ankle as indicated by 10. The pivot or hinge structure 14 may be further defined as a structure combining the upper half of the orthosis indicated by 12 to the lower half 13 and enabling rotation around a specific axis optionally with a certain predetermined resistance to rotation and/or optionally with a certain range of rotation (i.e. allowed angle of rotation). The pivot or hinge structure 14 is shown in a simplified form in Fig. 2B to better illustrate the relevant structures as will be described in more detail in further figures without presenting the whole orthotic device. The hinge structure 14 has a first part 15 (e.g. that will be part of or attached to the upper part 12 of the orthosis) and a second part 16 (e.g. that will be part of or attached to the lower part 13 of the orthosis). Between these two parts is a rotational hinge 17 enabling rotation around a specific axis optionally with a certain predetermined resistance to rotation and/or optionally with a certain range of rotation (i.e. allowed angle of rotation).

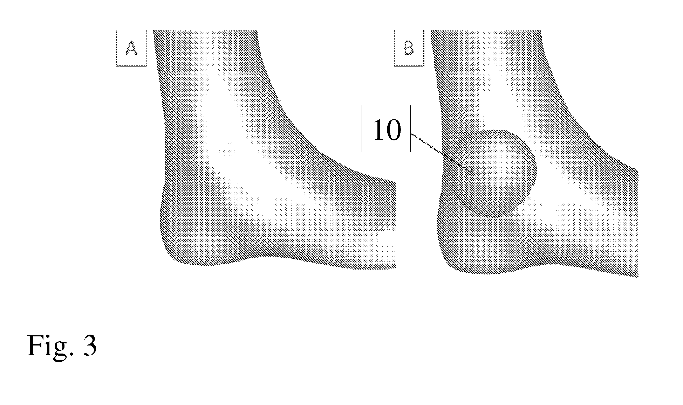

The axis of rotation for the hinge 17 in an orthotic device can be defined by a person skilled in the art from anatomical features and their location relation to each other and/or by physical examination, e.g. feeling/observing/measuring the movement of the joint in question (Fig. 3A). This axis of rotation, can be defined by a vector.

The axis of rotation may also used as the symmetry axis of the pivot or hinge structure 14 in order for the hinge 17 to be able to rotate around the defined axis. In one preferred embodiment, a curve that intersects with axis of rotation 10 is rotated around axis 10 at least by the angular amount of degrees needed to achieve the range of motion determined for the orthotic device. The resulting surface of revolution, as indicated by 10 in Fig 3B, may then be used as the inner surface of the hinge structure 14. The surface of revolution may be constructed from any arbitrary curve that intersects with the axis of rotation. Preferably, the curve is selected such that the hinge structure 14 can be placed as close to the ankle surface as possible providing that an offset is inserted between the hinge structure 14 and the ankle surface to maintain the ability to rotate freely. In a preferred embodiment the curve is defined as the cross section of the ankle outer surface and a plane through the defined axis of rotation. In another embodiment it can be an elliptical curve that approximates the shape of the ankle as in Fig. 3B.

The dimensions and location of the surface of revolution can be measured or approximated from whatever relevant parameter a person skilled in the art wants to determine, such as but not limited to the location and distance between the malleoli and/or other anatomical landmarks, type of pathology, amount of ankle pain, level of activity of the patient, type of footwear used by the patient.

Also, more than one surface of revolution or a combination of surfaces can be used. This type of offset curves can be seen useful for example around the knee, hip, elbow and/or wrist or any other relevant body part.

The range of motion for the pivot or hinge structure for a particular patient should be defined by a person skilled in the art in degrees from the "zero" position and may be dependent on e.g. the pathology and activity level of the patient. The hinges and hinge structures described with reference to embodiments of the present invention are able to provide suitable ranges of motion and to limit these ranges if required.

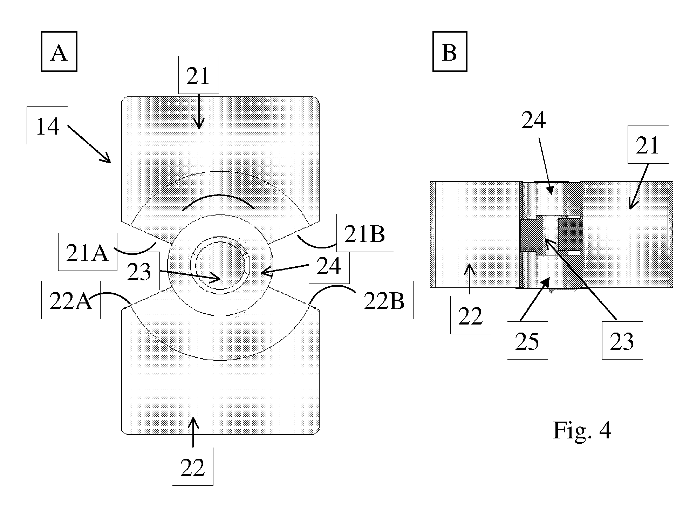

A first preferred embodiment of a hinge structure 14, referred to as a two-part hinge, is presented in Fig 4a and Fig. 4b (side view). As illustrated, the hinge structure 14 comprises of an upper part 21 and a lower part 22 rotating around a central axis 23 that is a part of the upper part 21. At the hinge, the lower part 22 is formed as two ring structures 24, 25 spaced apart along the same axis. The upper part 21 is connected to a shaft 23 which has a diameter and a length that it locates within the ring structures 24, 25. The range of rotation is limited by inclined faces 21a, b and 22 a, b of the upper and lower parts 21, 22 respectively. Such a part 14 can be made by rapid prototyping techniques. The hinge structure 14 can be used in an exoskeletal device such as an orthosis device comprising a first (21) and a second part (22), the first part being joined to the second part by the pivot or hinge structure 14 which allows pivotal rotational movement of the first part with respect to the second part. The pivot structure 14 can be formed integrally with the first and second part by a rapid prototyping technique. The pivot or hinge structure has a centre of pivoting about which the first and second parts can rotate with respect to each other. Although the hinge structure is shown flat the surface of the pivot structure can have a shape that deviates from planar, e.g. the surface being a surface of rotation, the centre of the surface of rotation coinciding with the centre of pivoting. A limiter of the angular rotational movement of the first part with respect to the second part can also be provided, e.g. formed integrally with the pivot structure by the rapid prototyping technique. Means for providing a resilient force resisting rotation of the first part with respect to the second part can also be included, e.g. formed integrally with the pivot structure by the rapid prototyping technique. The means for providing a resilient force can be provided a connection between the first and second parts as an elastically or plastically deformable structure.

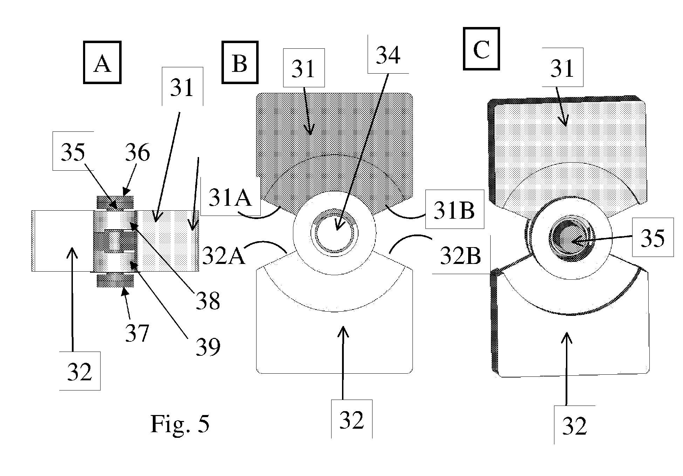

In another preferred embodiment presented in Fig 5, referred to as the three-part hinge 14, the upper part 31, lower part 32 and shaft 35 are all separate parts built together in the same hinge structure 14. In Fig. 5b, the shaft 35 is removed to illustrate the hole 34 in the upper part 31 to accommodate the shaft 35 shown in Fig. 5C. At the hinge, the lower part 32 is formed as two ring structures 38, 39 spaced apart along the same axis. The upper part 31 is connected to a tube that surrounds hole 34. The shaft 35 is preferably terminated by end disks 36 and 37 at each end to prevent the hinge structure 14 from falling apart. The range of rotation is limited by inclined faces 31a, b and 32 a, b of the upper and lower parts 31, 32 respectively. Such a part 14 can be made by rapid prototyping techniques. This hinge structure can also be used in an exoskeletal device such as an orthosis device comprising a first (31) and a second part (32), the first part being joined to the second part by the pivot or hinge structure which allows pivotal rotational movement of the first part with respect to the second part. The pivot structure can be formed integrally with the first and second part by a rapid prototyping technique. The pivot or hinge structure has a centre of pivoting about which the first and second parts can rotate with respect to each other. Although the hinge structure is shown flat the surface of the pivot structure can have a shape that deviates from planar, e.g. the surface being a surface of rotation, the centre of the surface of rotation coinciding with the centre of pivoting. A limiter of the angular rotational movement of the first part with respect to the second part can also be provided, e.g. formed integrally with the pivot structure by the rapid prototyping technique. Means for providing a resilient force resisting rotation of the first part with respect to the second part can also be included, e.g. formed integrally with the pivot structure by the rapid prototyping technique. The means for providing a resilient force can be provided a connection between the first and second parts as an elastically or plastically deformable structure.

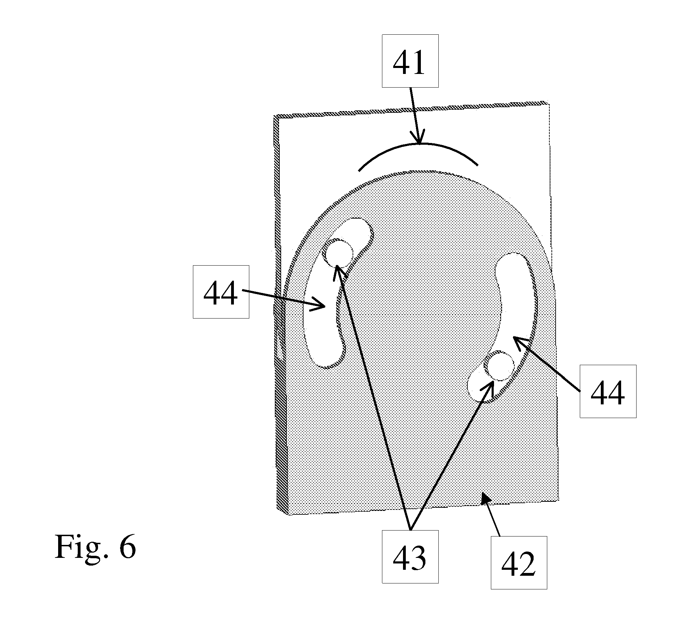

In Fig 6 yet another preferred embodiment of a part of a hinge structure 14 is presented that can be used with any of the embodiments of the present invention. The upper (21, 31....) and lower (22, 32.....) parts of a hinge structure 14 are extended to form at some position overlapping sections 41, 42. Part 41 can rotate with respect to part 42 using any of the hinges described with respect to any embodiment of the present invention. Two curved slots 44 are formed in one of parts 41, 42 (in part 42 as shown in Fig. 6) and two pins 43 are formed on the other of the parts 41, 42 (in part 41 as shown). The angle of rotation that is possible is limited by the two pins 43 moving in slots 44 until they reach the end of the slots. The hinge structure 14 can be used in an exoskeletal device such as an orthosis device comprising a first (41) and a second part (42), the first part being joined to the second part by the pivot or hinge structure 14 which allows pivotal rotational movement of the first part with respect to the second part. The pivot structure 14 can be formed integrally with the first and second part by a rapid prototyping technique. The pivot or hinge structure has a centre of pivoting about which the first and second parts can rotate with respect to each other. Although the hinge structure is shown flat the surface of the pivot structure can have a shape that deviates from planar, e.g. the surface being a surface of rotation, the centre of the surface of rotation coinciding with the centre of pivoting. Means for providing a resilient force resisting rotation of the first part with respect to the second part can also be included, e.g. formed integrally with the pivot structure by the rapid prototyping technique. The means for providing a resilient force can be provided a connection between the first and second parts as an elastically or plastically deformable structure.

To enable the additive fabrication of any or all of these structures in figures 4 to 6, with a layered manufacture, e.g. rapid prototyping technique for example the SLS process, in any form of artificial exoskeletal devices and/or machines, orthotic devices and, more specifically, artificial exoskeletal devices and/or machines, orthotic devices having a pivot or hinge structure integrated into the main body of the artificial exoskeleton device and/or machines, or orthosis as well as methods for computer aided designing and making of these devices, gaps must be left between the moving surfaces so that the surfaces do not melt together and any rest materials such as unsintered powder can be cleaned from between the surfaces. Preferably, a gap in the range of 0.1-1.5mm is chosen. The specific form of the hinge structure can vary. The dimensions of the different structures (such as thickness, wall thicknesses, specific dimensions and radiuses) can be modified to match the patient and the application.

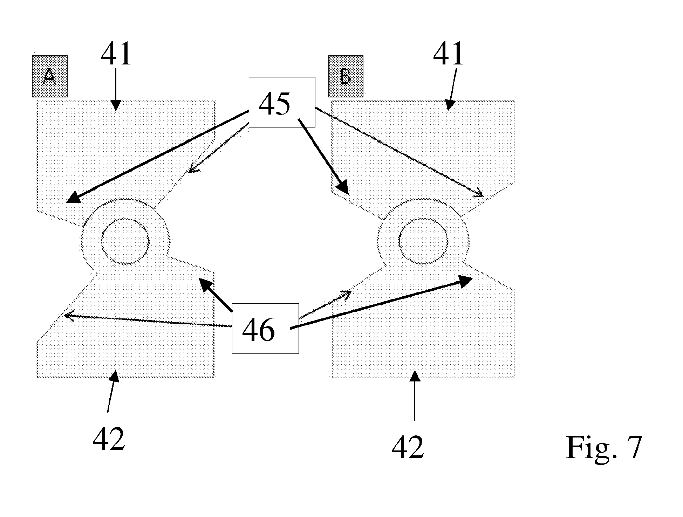

The pivot or hinge structure according to any embodiment of the present invention such as used in artificial exoskeletal devices and/or machines, orthotic devices and, more specifically, in artificial exoskeletal devices and/or machines, orthotic devices having the pivot or hinge structure integrated into the main body of the artificial exoskeleton device and/or machines, or orthosis as well as in methods for computer aided designing and making of these devices, may also comprise at least one set of edge structures 45 and 46, as illustrated on Fig. 7A and Fig. 7B. Edge structures 45 and 46 are formed by a layer manufacturing technique such as a rapid prototyping technique and physically limit the range of motion of the hinge. In Fig. 7B B, a different range is enabled compared to Fig. 7A by a different position of the edge structures 45 and 46. This type of limited range of motion can be designed during the CAD design of the hinge. Similar means of controlling the range of motion in other embodiments are within the scope of this invention. The hinge structure can be used in an exoskeletal device such as an orthosis comprising a first (41) and a second part (42), the first part being joined to the second part by the pivot or hinge structure which allows pivotal rotational movement of the first part with respect to the second part. The pivot structure can be formed integrally with the first and second part by a rapid prototyping technique. The pivot or hinge structure has a centre of pivoting about which the first and second parts can rotate with respect to each other. Although the hinge structure is shown flat the surface of the pivot structure can have a shape that deviates from planar, e.g. the surface being a surface of rotation, the centre of the surface of rotation coinciding with the centre of pivoting. Means for providing a resilient force resisting rotation of the first part with respect to the second part can also be included, e.g. formed integrally with the pivot structure by the rapid prototyping technique. The means for providing a resilient force can be provided a connection between the first and second parts as an elastically or plastically deformable structure.

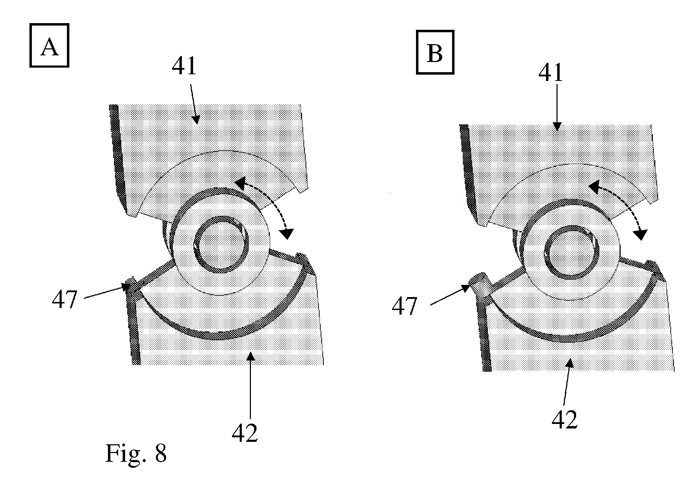

In another embodiment, the range of motion may be adjusted e.g. by placing a blocking device such as a screw 47 in a relevant location, as illustrated in Fig 8A, where 47 indicates a screw or a screw-like structure that can be embedded into one or both sides of the hinge structure. As the screw 47 is rotated, it moves and alters the range of motion as indicated in Fig. 8 B. The screw 47 itself can move or it can push a pin, a wedge or a similar structure in place. Similar means of controlling the range of motion in other embodiments are also included in the scope of this invention. This hinge structure can also be used in an exoskeletal device such as an orthosis comprising a first (41) and a second part (42), the first part being joined to the second part by the pivot or hinge structure which allows pivotal rotational movement of the first part with respect to the second part. The pivot structure can be formed integrally with the first and second part by a rapid prototyping technique. The pivot or hinge structure has a centre of pivoting about which the first and second parts can rotate with respect to each other. Although the hinge structure is shown flat the surface of the pivot structure can have a shape that deviates from planar, e.g. the surface being a surface of rotation, the centre of the surface of rotation coinciding with the centre of pivoting. Means for providing a resilient force resisting rotation of the first part with respect to the second part can also be included, e.g. formed integrally with the pivot structure by the rapid prototyping technique. The means for providing a resilient force can be provided a connection between the first and second parts as an elastically or plastically deformable structure.

The pivot or hinge structure in an orthotic device, including, but not limited to, knee orthoses, knee-ankle-foot orthoses, hip orthoses, hip-knee-ankle-foot-orthosis, lumbar orthoses,adaptors or joints and cranial helmets can also comprise at least one spring structure, having a spring coefficient (**k** ), e.g. measured in Newton-meters/radian, controlling the resistance to rotation connecting said upper part of the hinge structure and said lower part of the hinge structure or, in case of a three-part hinge structure, connecting said upper part and said lower part of the hinge structure to said central axis. Said spring structure may comprise beam shaped structures that elastically deform. This elastic or plastic deformation will resist the ankle rotation α with the moment M in Newton-meters. This can be expressed as M=k α. For multiple springs, these can be added, for example two different springs would resist the motion with a moment M₁ + M₂ = (k₁ + k₂) α. For hinges with multiple spring types the k and α values can vary depending on the elastic deformation of the whole overall structure and in complex cases, more sophisticated computational methods, such as Finite Element analysis may be required.

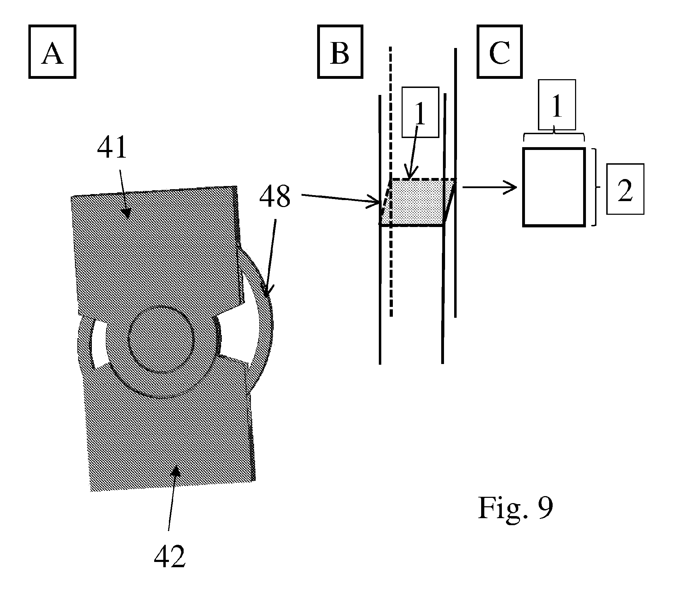

In one preferred embodiment, according to FIG 9, the spring structure is a coil spring as indicated by 48 in Fig. 9A which is located between the first and second parts 41 and 42 of the hinge structure. The cross-section of the springs as indicated by 48 in Fig. 9 B may also be adjusted to change the resistance to deformation. Different k values may be achieved by thickening the spring in any or all directions, such as 1 and/or 2 in Fig. 9C. This thickening can be introduced in a layered manufacturing technique such as a rapid prototyping technique. Also, the length of the spring maybe adjusted individually or in combination with other modifications. This hinge structure can also be used in an exoskeletal device such as an orthosis comprising a first (41) and a second part (42), the first part being joined to the second part by the pivot or hinge structure which allows pivotal rotational movement of the first part with respect to the second part. The pivot structure can be formed integrally with the first and second part by a rapid prototyping technique. The pivot or hinge structure has a centre of pivoting about which the first and second parts can rotate with respect to each other. Although the hinge structure is shown flat the surface of the pivot structure can have a shape that deviates from planar, e.g. the surface being a surface of rotation, the centre of the surface of rotation coinciding with the centre of pivoting. A limiter of the angular rotational movement of the first part with respect to the second part can also be provided, e.g. formed integrally with the pivot structure by the rapid prototyping technique.

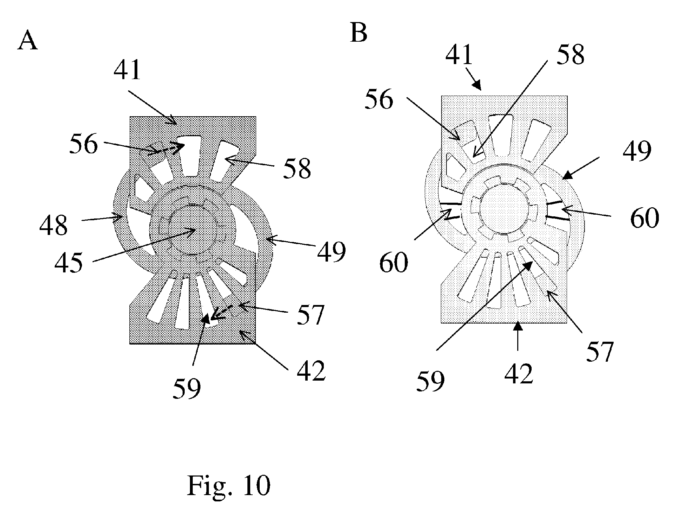

Another spring can also be added inside the hinge structure space permitting, such as shown with reference numbers 48 and 49 in Fig 10A and B. The springs 48, 49 are located between the first and second parts 41 and 42 of the hinge structure. The enlarged ends 56, 57 of springs 48, 49 may locate into spaces 58, 59 in parts 41 and 42 respectively. The spaces 58, 59 are arranged circumferentially in the parts 41, 52 thus allowing different positions for placement of the enlarged ends 56; 57. Preferably, the cross-section of a springs 48 49 has a rectangular shape, but may have any other arbitrary shape including but not limited to a circular, triangular, semi-circular, elliptical, hexagonal or any combination thereof. The length of the springs can be set by selecting the points where it connects to the upper and lower halves accordingly, i.e. in which spaces 58, 59 the enlarged ends 56, 57 are inserted. The spaces 58, 59 in which the enlarged ends 56, 57 can be inserted can be changed manually to provide a different spring function. In Fig. 10B additional braces 60 are provided to alter the spring constants of springs 48, 49. Braces 60 can be made removable. This hinge structure can also be used in an exoskeletal device such as an orthosis comprising a first (41) and a second part (42), the first part being joined to the second part by the pivot or hinge structure which allows pivotal rotational movement of the first part with respect to the second part. The pivot structure can be formed integrally with the first and second part by a rapid prototyping technique. The pivot or hinge structure has a centre of pivoting about which the first and second parts can rotate with respect to each other. Although the hinge structure is shown flat the surface of the pivot structure can have a shape that deviates from planar, e.g. the surface being a surface of rotation, the centre of the surface of rotation coinciding with the centre of pivoting. A limiter of the angular rotational movement of the first part with respect to the second part can also be provided, e.g. formed integrally with the pivot structure by the rapid prototyping technique.

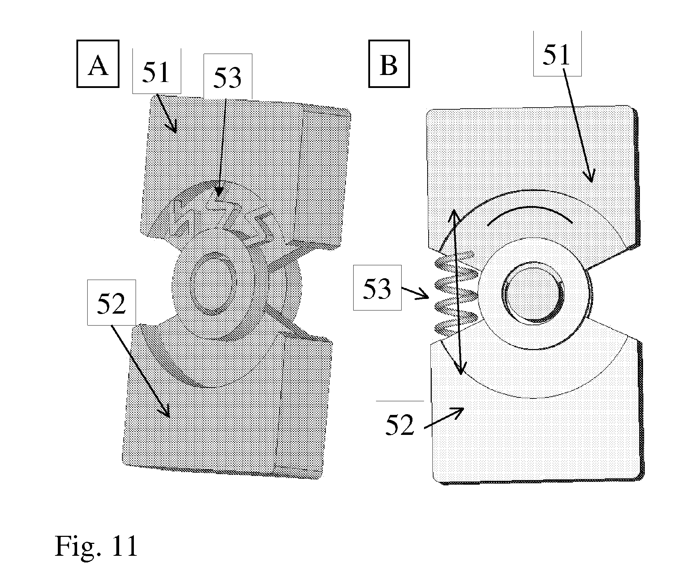

Another preferred embodiment is presented in Fig 11, where the upper and lower parts of the pivot or hinge structure as indicated by 51 and 52 in Fig. 11 A, are connected by one or more beam structures 53, where the beam or beams deform in response to the rotation of the hinge. These beams 53 can be a beam that is straight or has one or more angles before connecting to the other side of the hinge or one or more curved sections with certain radiuses or any combination thereof. The thickness of the beam 53 can also be varied as necessary and the beams can also be hollow. Another specific embodiment is the spiral spring 53 in Fig. 11 B, where one or more spiral springs 53 are used to provide resistance to the hinge rotation. The spiral springs 53 may be round or elliptical, have a certain thickness, length, radius and number of revolutions around a longitudinal axis. All of these properties may be modified to match the required k values. These hinge structures can also be used in an exoskeletal device such as an orthosis comprising a first (51) and a second part (52), the first part being joined to the second part by the pivot or hinge structure which allows pivotal rotational movement of the first part with respect to the second part. The pivot structure can be formed integrally with the first and second part by a rapid prototyping technique. The pivot or hinge structure has a centre of pivoting about which the first and second parts can rotate with respect to each other. Although the hinge structure is shown flat the surface of the pivot structure can have a shape that deviates from planar, e.g. the surface being a surface of rotation, the centre of the surface of rotation coinciding with the centre of pivoting. A limiter of the angular rotational movement of the first part with respect to the second part can also be provided, e.g. formed integrally with the pivot structure by the rapid prototyping technique.

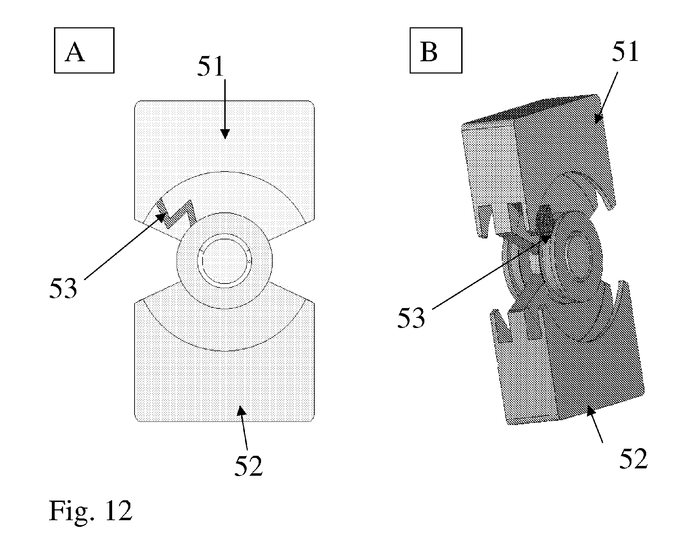

Another preferred embodiment is presented in Fig. 12, where the upper and lower parts of the pivot or hinge structure as indicated by 51 and 52 are similar to Fig. 11 B, and are connected by one or more beam structures 53, where the beam or beams deform in response to the rotation of the hinge. These beams 53 can be a beam that is straight or has one or more angles before connecting to the other side of the hinge or one or more curved sections with certain radiuses or any combination thereof. The thickness of the beam 53 can also be varied as necessary and the beams can also be hollow. As shown in Fig. 12 B, the beam or beams 53 can be included In a groove formed in the first and second parts 51 and 52. All of the properties of the beams 53 may be modified to match the required k values. These hinge structures can also be used in an exoskeletal device such as an orthosis comprising a first (51) and a second part (52), the first part being joined to the second part by the pivot or hinge structure which allows pivotal rotational movement of the first part with respect to the second part. The pivot structure can be formed integrally with the first and second part by a rapid prototyping technique. The pivot or hinge structure has a centre of pivoting about which the first and second parts can rotate with respect to each other. Although the hinge structure is shown flat the surface of the pivot structure can have a shape that deviates from planar, e.g. the surface being a surface of rotation, the centre of the surface of rotation coinciding with the centre of pivoting. A limiter of the angular rotational movement of the first part with respect to the second part can also be provided, e.g. formed integrally with the pivot structure by the rapid prototyping technique.

The k value of the springs 53 can be adjusted for each patient by picking a hinge structure corresponding to the intended k value range from a library in the CAD design system. The k value or k value range needed for each patient is determined by the clinician after assessing the patient. The library may consist of different types of spring structures, such as those presented in figures 9, 10, 11 and 12 - and the performance values and/or other practical considerations linked to those structures, such as the k values, physical dimensions of the spring and/or the surrounding structure limiting the range of motion, size and location of any holes for powder clearance or any other relevant property of the structure.

In another embodiment, hollow sections inside the deformable structure can be pressurized with air or a liquid increasing the tension in the structure stiffening it and increasing the resistance to deformation in the structure.

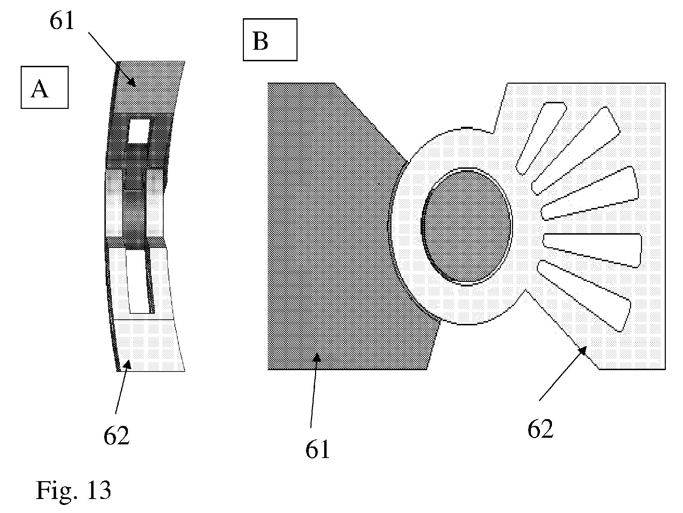

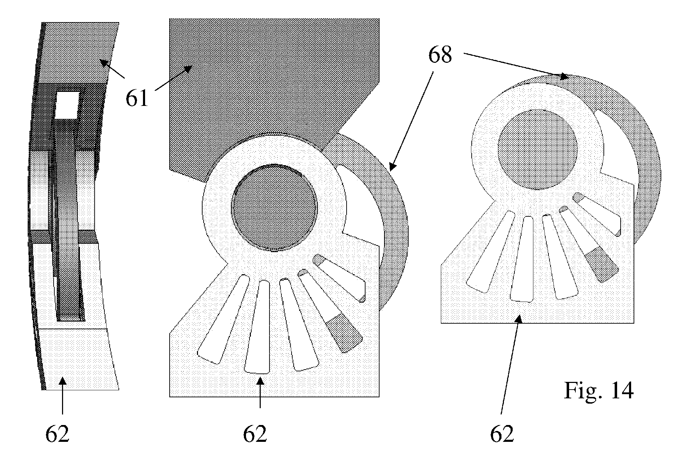

As indicated above any of the hinge structures described need not be planar. As shown in Figs. 13 and 14 in any embodiment upper and lower parts of the pivot or hinge structure are indicated by 61 and 62. These rotate with respect to each other and the surface of the pivot structure can have a shape that deviates from planar, e.g. the surface being a surface of rotation, the centre of the surface of rotation coinciding with the centre of pivoting. Such a surface can be provided for any of the embodiments of the invention and is suitable for providing close fitting hinges to joints such as knees, ankles elbows etc. The upper and lower parts 61, 62 of the pivot or hinge structure can be connected by one or more springs or beam structures 68, where the beam or beams deform in response to the rotation of the hinge. These beams 68 can be a beam that is curved, helical, spiral, straight or has one or more angles before connecting to the other side of the hinge or one or more curved sections with certain radiuses or any combination thereof. The thickness of the beam 68 can also be varied as necessary and the beams can also be hollow. All of the properties of the beam 68 may be modified to match the required k values. These hinge structures can also be used in an exoskeletal device such as an orthosis comprising a first (61) and a second part (62), the first part being joined to the second part by the pivot or hinge structure which allows pivotal rotational movement of the first part with respect to the second part. The pivot structure can be formed integrally with the first and second part by a rapid prototyping technique. The pivot or hinge structure has a centre of pivoting about which the first and second parts can rotate with respect to each other. A limiter of the angular rotational movement of the first part with respect to the second part can also be provided, e.g. formed integrally with the pivot structure by the rapid prototyping technique.

In any of the embodiments of the present invention the pivot or hinge structure can be rotated by means of a drive mechanism, e.g. via the means of a motor and/or actuator and the elastic deformation in the structure can also provide the force to move the pivot or hinge structure back to its original position.

In another embodiment, cylindrical hinge structures are used as well, but the other end of the deforming spring structure is attached on the upper half of the hinge structure and the other to the lower. The spring structure can then be built inside the upper or lower halves, or be external connections on the outside.

The specific properties of the hinge structure used for a particular patient, such as range of motion, resistance to rotation or any other meaningful property may be quantified experimentally or by using analysis methods, such as finite element analysis. The specific mechanical requirement for the structure for a particular patient can then be linked to that patient by the means of gait analysis, pressure or force measurements, and manual estimation by a clinician or any combination thereof.

In the preferred embodiment the CAD system will place the pivot or hinge structures of any of the embodiments of the present invention to the ideal location and the designer can then accept or modify the proposed design in any arbitrary way.

Such a design method is a computer based method. The present invention also provides a computer program product comprising code segments, the code segments comprising, when executed on a computing device: means for receiving scan data describing at least part of the patient body; means for converting said scan data into a 3D virtual model on the basis of which a 3D design model of the orthotic device may be constructed; and means for adding automatically or interactively at least one hinge structure and it's an adjustment mechanism enabling means for adjusting at least one meaningful property of said hinge from a library of hinge and adjustment structures; and fabricating the orthotic device.

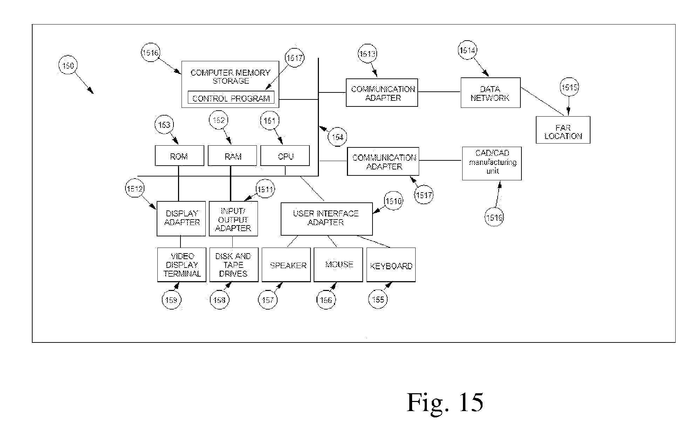

FIG. 15 is a schematic representation of a computing system which can be utilized with the methods and in a system according to the present invention including computer programs such as 3-matic™ as supplied by Materialise N.V., Leuven, Belgium. A computer 150 is depicted which may include a video display terminal 159, a data input means such as a keyboard 155, and a graphic user interface indicating means such as a mouse 156. Computer 150 may be implemented as a general purpose computer, e.g. a UNIX workstation or a personal computer.

Computer 150 includes a Central Processing Unit ("CPU") 151, such as a conventional microprocessor of which a Pentium processor supplied by Intel Corp. USA is only an example, and a number of other units interconnected via bus system 154. The bus system 154 may be any suitable bus system - FIG. 15 is only schematic. The computer 150 includes at least one memory. Memory may include any of a variety of data storage devices known to the skilled person such as random-access memory ("RAM"), read-only memory ("ROM"), non-volatile read/write memory such as a hard disc as known to the skilled person. For example, computer 150 may further include random-access memory ("RAM") 152, read-only memory ("ROM") 153, as well as a display adapter 1512 for connecting system bus 154 to a video display terminal 159, and an optional input/output (I/O) adapter 1511 for connecting peripheral devices (e.g., disk and tape drives 158) to system bus 154. Video display terminal 159 can be the visual output of computer 150, which can be any suitable display device such as a CRT-based video display well-known in the art of computer hardware. However, with a desk-top computer, a portable or a notebook-based computer, video display terminal 159 can be replaced with a LCD-based or a gas plasma-based flat-panel display. Computer 150 further includes user interface adapter 1510 for connecting a keyboard 155, mouse 156, optional speaker 157. The relevant data describing the 3-D object to be formed may be input directly into the computer using the keyboard 155 or from storage devices such as 158, after which a processor carries out a method in accordance with the present invention. The results of the method may be transmitted to a further near or remote location, e.g. a CAD/CAM processing facility to manufacture the orthotic device in accordance with the details provided by computer 150.

A CAD/CAM manufacturing unit 1516 may also be connected via a communications adapter 1517 to bus 154 connecting computer 150 to a data network such as the Internet, an Intranet a Local or Wide Area network (LAN or WAN) or a CAN. The manufacturing unit 1516 may receive an output value or support descriptor file directly from computer 150 running a computer program for support design in accordance with the present invention or a value or descriptor file derived from such an output of computer 150. Alternatively, the unit 1516 may receive the relevant design data indirectly on a suitable signal storage medium such as a diskette, a replaceable hard disc, an optical storage device such as a CD-ROM or DVD-ROM, a magnetic tape or similar.

Computer 150 also includes a graphical user interface that resides within machine-readable media to direct the operation of computer 150. Any suitable machine-readable media may retain the graphical user interface, such as a random access memory (RAM) 152, a read-only memory (ROM) 153, a magnetic diskette, magnetic tape, or optical disk (the last three being located in disk and tape drives 158). Any suitable operating system and associated graphical user interface (e.g., Microsoft Windows, Linux) may direct CPU 151. In addition, computer 150 includes a control program 1517 that resides within computer memory storage 1516. Control program 1517 contains instructions that when executed on CPU 151 allow the computer 150 to carry out the operations described with respect to any of the methods of the present invention.

Those skilled in the art will appreciate that the hardware represented in FIG. 15 may vary for specific applications. For example, other peripheral devices such as optical disk media, audio adapters, or chip programming devices, such as PAL or EPROM programming devices well-known in the art of computer hardware, and the like may be utilized in addition to or in place of the hardware already described.

In the example depicted in FIG. 15, the computer program product for carrying out the method of the present invention can reside in any suitable memory. However, it is important that while the present invention has been, and will continue to be, that those skilled in the art will appreciate that the mechanisms of the present invention are capable of being distributed as a computer program product in a variety of forms, and that the present invention applies equally regardless of the particular type of signal bearing media used to actually carry out the distribution. Examples of computer readable signal bearing media include: recordable type media such as floppy disks and CD ROMs and transmission type media such as digital and analogue communication links.

Accordingly, the present invention also includes a software product which when executed on a suitable computing device carries out any of the methods of the present invention. Suitable software can be obtained by programming in a suitable high level language such as C and compiling on a suitable compiler for the target computer processor.

Having designed the orthotic device, it can be manufactured, as illustrated as module 6 in FIG. 1. In one preferred embodiment, Rapid Prototyping and Manufacturing (RP&M) techniques are used to manufacture the device. Rapid Prototyping and Manufacturing (RP&M) can be defined as a group of techniques used to quickly fabricate a scale model of an object typically using three-dimensional (3-D) computer aided design (CAD) data of the object. Currently, a multitude of Rapid Prototyping techniques is available, including stereo lithography (SLA), Selective Laser Sintering (SLS), Fused Deposition Modeling (FDM), foil-based techniques, etc.

A common feature of these techniques is that objects are typically built layer by layer. Stereo lithography, presently the most common RP&M technique, utilizes a vat of liquid photopolymer "resin" to build an object a layer at a time. On each layer, an electromagnetic ray, e.g. one or several laser beams which are computer-controlled, traces a specific pattern on the surface of the liquid resin that is defined by the two-dimensional cross-sections of the object to be formed. Exposure to the electromagnetic ray cures, or, solidifies the pattern traced on the resin and adheres it to the layer below. After a coat had been polymerized, the platform descends by a single layer thickness and a subsequent layer pattern is traced, adhering to the previous layer. A complete 3-D object is formed by this process.

Selective laser sintering (SLS) uses a high power laser or another focused heat source to sinter or weld small particles of plastic, metal, or ceramic powders into a mass representing the 3-dimensional object to be formed.

Fused deposition modeling (FDM) and related techniques make use of a temporary transition from a solid material to a liquid state, usually due to heating. The material is driven through an extrusion nozzle in a controlled way and deposited in the required place as described among others in U.S. Pat. No. 5.141.680.

Foil-based techniques fix coats to one another by means of gluing or photo polymerization or other techniques and cut the object from these coats or polymerize the object. Such a technique is described in U.S. Pat. No. 5.192.539.

Typically RP&M techniques start from a digital representation of the 3-D object to be formed. Generally, the digital is sliced into a series of cross-sectional layers which can be overlaid to form the object as a whole. The RP&M apparatus uses this data for building the object on a layer-by-layer basis. The cross-sectional data representing the layer data of the 3-D object may be generated using a computer system and computer aided design and manufacturing (CAD/CAM) software.

A selective laser sintering (SLS) apparatus is particularly preferred for the manufacture of the orthotic device from a computer model. It should be understood however, that various types of rapid manufacturing and tooling may be used for accurately fabricating these orthotic devices including, but not limited to, stereolithography (SLA), Fused Deposition Modeling (FDM) or milling.

The orthotic device may be manufactured in different materials. Preferably, only materials that are biocompatible with the human body are taken into account. In the case SLS is used as a RP&M technique, the orthotic device may be fabricated from a polyamide such as PA 2200 as supplied by EOS, Munich, Germany or Duraform PA from 3D Systems, South Caroline, USA, or any other material known by those skilled in the art may also be used. The orthotic may also be painted and/or coated using any suitable means.

The present invention may provide one or more of the following advantages:
● The quality of the devices is more consistent as significant manual intervention is no longer needed.
● Less labor is needed. The labor does not need to be as skilled in all of the manual work phases. Only in scanning, CAD design, finishing and fitting the parts.
● Less production equipment may be needed (one machine).
● The current invention may result in less waste from the manufacturing process.
● The current invention may result in a faster production.
● The current invention may result in more automated production.
● With the current invention, there is no longer a need to store casts as everything can be stored digitally and reproduced as needed. Also, the whole orthotic treatment history of the patient is available for clinicians to use when needed.

## Claims

1. An exoskeletal device wherein the exoskeletal device is an orthosis comprising a first and a second part, the first part being joined to the second part by a pivot structure which allows pivotal rotational movement of the first part with respect to the second part, wherein the pivot structure is formed integrally with the first and second part by a rapid prototyping technique, the device being formed as a shell for encompassing a limb of an animal or human, the pivot structure having a centre of pivoting about which the first and second parts can rotate with respect to each other, a surface of the pivot structure having a shape that deviates from planar, the surface being a surface of rotation, the centre of the surface of rotation coinciding with the centre of pivoting.

2. The device of claim 1, further comprising a means for limiting the angular rotational movement of the first part with respect to the second part, the means for limiting being optionally formed integrally with the pivot structure by the rapid prototyping technique.

3. The device of any previous claim, further comprising means for providing a resilient force resisting rotation of the first part with respect to the second part.

4. The device of claim 3, wherein the means for providing a resilient force is formed integrally with the pivot structure by the rapid prototyping technique and/or wherein the means for providing a resilient force comprises a connection between the first and second parts having an elastically or plastically deformable structure and/or wherein the means for providing a resilient force is adjustable.

5. The device of claim 4, wherein the elastically or plastically deformable structure is in the form of a spiral or coil.

6. The device according to any previous claim wherein the pivot structure is a hinge.

7. A method of forming an exoskeletal device, wherein the exoskeletal device is an orthosis comprising a first and a second part, the first part being joined to the second part by a pivot structure which allows pivotal rotational movement of the first part with respect to the second part, the method comprising forming the pivot structure integrally with the first and second part by a rapid prototyping technique, as a shell for encompassing a limb of an animal or human, the pivot structure having a centre of pivoting about which the first and second parts can rotate with respect to each other, a surface of the pivot structure having a shape that deviates from planar, the surface being a surface of rotation, the centre of the surface of rotation coinciding with the centre of pivoting.

8. The method of claim 8, further comprising forming a limiter of the angular rotational movement of the first part with respect to the second part, the limiter being optionally formed integrally with the pivot structure by the rapid prototyping technique.

9. The method of any of the claims 7 or 8, further comprising forming means providing a resilient force resisting rotation of the first part with respect to the second part.

10. The method of claim 9, wherein the means for providing a resilient force is formed integrally with the pivot structure by the rapid prototyping technique, and/or forming the means for providing a resilient force as a connection between the first and second parts as an elastically or plastically deformable structure.

11. The method of claim 10, wherein the elastically or plastically deformable structure is formed as a spiral or coil.

12. A computer based method of forming an orthotic device for a patient comprising the steps of:
receiving scan data describing at least part of the patient's body;
converting said scan data into a 3D virtual model on the basis of which a 3D design model of the orthotic device is constructed;
adding automatically or interactively at least one pivot structure from a library of pivot structures, the pivot structure having a centre of pivoting about which the first and
second parts can rotate with respect to each other, a surface of the pivot structure having a shape that deviates from planar, the surface being a surface of rotation, the centre of the surface of rotation coinciding with the centre of pivoting;
and
fabricating the orthotic device.

13. The method of claim 12, wherein the adding step includes adding an adjustment mechanism for adjusting at least one meaningful property of said pivot structure from a library of adjustment structures.

14. The method of claim 12 or 13, further comprising:
manufacturing a first and a second part of the orthotic device,
manufacturing the pivot structure so that the first part being is joined to the second part by the pivot structure which allows pivotal rotational movement of the first part with respect to the second part, the pivot structure being formed integrally with the first and second part by a rapid prototyping technique.

15. A computer program product comprising code segments, the code segments comprising, when executed on a computing device:
means for receiving scan data describing at least part of the patient body;
means for converting said scan data into a 3D virtual model on the basis of which a 3D design model of the orthotic device may be constructed; and
means for adding automatically or interactively at least one pivot structure and an adjustment mechanism enabling means for adjusting at least one meaningful property of said pivot structure from a library of pivot and adjustment structures.
